# EUROPÄISCHE PATENTSCHRIFT

(11) **EP 1 235 710 B1**
(45) Veröffentlichungstag und Bekanntmachung des Hinweises auf die Patenterteilung: **10.09.2003**
(21) Anmeldenummer: 00985115.5
(22) Anmeldetag: 29.11.2000
(51) Int. Cl.: B63C 11/14, A62B 7/12, A61B 18/02, A61M 16/06

(54) **MUND/NASE-FRISCHLUFT-MASKE**
MOUTH/NOSE FRESH AIR MASK
MASQUE A AIR FRAIS POUR LA BOUCHE ET/OU LE NEZ

(30) Priorität: 29.11.1999 DE 19957497
(43) Veröffentlichungstag der Anmeldung: 04.09.2002
(73) Patentinhaber: Pfeiffer, Maria, 97299 Zell a. Main (DE)
(72) Erfinder: Pfeiffer, Maria, 97299 Zell (DE); Klammes, Rolf, 97209 Veitschöchheim (DE)
(74) Vertreter: Leske, Thomas, Dr.
(86) Internationale Anmeldenummer: EP0011971
(87) Internationale Veröffentlichungsnummer: WO01040050

(56) Entgegenhaltungen:
- EP-A- 0 467 362
- WO-A-97/32619
- DE-A- 1 784 487
- FR-A- 2 053 375
- US-A- 2 875 756
- US-A- 5 404 873

## Beschreibung

Die vorliegende Erfindung betrifft eine Atemmaske zur Versorgung von Personen mit insbesondere Frischluft über Mund und/oder Nase, umfassend eine vollständig fluiddichte Grundmaske, die mit einem flexiblen Koaxialschlauch in Verbindung steht.

Genauer gesagt dient die Erfindung zur uneingeschränkten Frischluftversorgung in einem, zum freien Atmen ungeeigneten Medium bzw. zur Atemluftreinhaltung von Räumen in Freizeit, Beruf, Rettung und zum medizinisch/therapeutischen Einsatz, vorzugsweise unter Wasser.

Das Dokument DE 1 784 487 A beschreibt eine Atemmaske entsprechend dem Oberbegriff des Anspruchs 1.

Der Frischluftschnorchel gemäß DE 691 07 056 T2 bzw. EP 0509 016 B1 ermöglicht das Einatmen von Frischluft in Wasser, erlaubt jedoch nur eine Tauchtiefe von ca. 60 cm, was eine erhebliche Bewegungseinschränkung mit sich bringt. Der Schnorchel ist nur mit einem Mundstück ausgerüstet, was bewirkt, daß der Benutzer gezwungen ist, nur durch den Mund zu atmen, also eine freie Wahl des Atemvorgangs nicht möglich ist. Ebenso muß er darauf achten, daß er das Mundstück nicht durch Öffnen des Mundes verliert. Einer ins Koma gefallenen Person ist eine solche Kontrolle nicht möglich.

Die in der DE 197 27 640 A1 beschriebene Anordnung zur Versorgung von Personen mit Atemgas ist darauf gerichtet, daß eine bzw. mehrere bettlägerige Personen mittels mit Sauerstoff angereicherter Luft stationär versorgt werden sollen.

Diese zentrale Versorgung ist teuer und aufwendig. Ein weiterer Nachteil sind lange Leitungswege die mit entsprechendem Druck zur Aufhebung des Reibungswiderstandes versehen werden müssen. Die unter DE 197 27 640 A1 aufgeführte Atemmaske eignet sich nicht zum Einsatz unter Wasser und ist auch für einen Einsatz am gesunden Menschen in Arbeitsbereichen nicht geeignet.

Die in der Patentschrift DE 197 38 266 A1 beschriebene Steuerung, Regelung und Optimierung von Luftaustrittsöffnungen aller zur Behandlung von Schlafapnoe eingesetzten Atemmasken zur gezielten Ablenkung der Atem- und Überdrnckluft ist für den Einsatz unter Wasser nicht geeignet, da die Luftaustrittsöffnungen das Wasser in die Maske eintreten lassen würde.

Die DE 40 23 108 C1 bezieht sich auf ein Beatmungsbesteck zur Behandlung bzw. Narkotisierung von Patienten bzw. zur Beatmung von Patienten mit Atemproblemen. Aufgrund der Anordnung der Zusatzgeräte ist die Nutzung unter Wasser nicht möglich. Die Schlauchlänge der Frischluftzufuhr ist begrenzt. Zudem entstehen durch den direkten Anschluß an die Atemmaske unangenehme Zugerscheinungen, die eine physische und psychische Entspannung unmöglich machen.

Weiterhin bekannt sind Masken bei Schlafapnoegeräten, welche mit kleinen Öffnungen versehen sind und sich somit nicht in zum Atmen ungeeigneten Medium verwenden lassen.

Bekannte Masken im Anästhesiebereich müssen mit der Hand festgehalten werden und lassen somit dem Benutzer nicht die nötige Bewegungsfreiheit.

Taucherausrüstungen sind zum einen teuer, sehr unhandlich, schwer und nicht ohne größere Vorbereitung einsetzbar. Sauerstoffflaschen haben nur eine begrenzte Aufnahmefähigkeit. Reparaturen und dergleichen unter Wasser sind derzeit nur mit einer Taucherausrüstung ausführbar. Dies hat eine zeitliche Einschränkung zur Folge.

Der vorliegenden Erfindung lag somit die Aufgabe zugrunde, eine Atemmaske bereitzustellen, welche einen weitestgehend uneingeschränkten und entspannten Aufenthalt in einem zum ungeschützten Atmen ungeeigneten Medium ermöglicht und beispielsweise Reinsträume vor ausgeatmeter Luft schützt sowie einfach und kostengünstig herstellbar ist.

Diese Aufgabe wird erfindungsgemäß durch eine Atemmaske zur Versorgung von Personen mit insbesondere Frischluft über Mund und/oder Nase, umfassend eine vollständig fluiddichte Grundmaske, die mit einem flexiblen Koaxialschlauch in Verbindung steht, dadurch gekennzeichnet, daß zwischen dem flexiblen Koaxialschlauch und der Grundmaske eine Vorkammer angeordnet ist, wobei ein innerer Schlauch des Koaxialschlauchs mit einem in der Vorkammer vorgesehenen gebogenen Rohr verbunden ist und ein äußerer Schlauch des Koaxialschlauchs gegen die Vorkammer abgedichtet in diese mündet, gelöst.

Vorteilhafte und bevorzugte Ausgestaltungen der Erfindung sind in den Unteransprüchen angegeben.

Die Mund/Nase-Frischluft-Maske ist mittels eines flexiblen Koaxialschlauchs in notwendiger Länge mit dem Frischluft-führenden Bereich verbunden. Mittels Überdruck in geeigneter Stärke wird Frischluft oder ein zum atmen geeignetes Gas in einem der beiden Schläuche, vorzugsweise dem inneren Schlauch, zur Maske befördert. Dieser Schlauch mündet in einer kleinen Vorkammer vor der Grundmaske. Diese Vorkammer weist ein gebogenes Anschlußrohr für die Frischluftzufuhr auf, so daß es zu keinen störenden Zugempfindungen kommt. Gleichzeitig ist dadurch gewährleistet, daß die ausgeatmete Luft auf schnellstem Wege nach außen befördert wird (Totraumventilation). Somit ist gewährleistet, daß der Benutzer keine verbrauchte Luft rückatmet.

Im zweiten, vorzugsweise äußeren Schlauch, wird die verbrauchte Atemluft nach außen befördert, was den Vorteil hat, daß der Benutzer nicht durch unangenehme Blubbergeräusche der ausgeatmeten Luft in der Entspannung gestört wird. Die Benutzungsdauer der Mund/Nase-Frischluft-Maske ist hinsichtlich der Frischluft uneingeschränkt.

Die Mund/Nase-Frischluft-Maske ist mittels mehrer Klettbänder um den Kopf so fixierbar, daß sie nicht verrutschen kann und somit dem Benutzer volle Bewegungsfreiheit läßt. Da die Maske den Mund- und Nasenbereich gleichzeitig abdeckt, ist es nicht notwendig auf die Atmung zu achten, was dem Benutzer eine optimale Entspannung ermöglicht. Es ist vollkommen egal, ob mit dem Mund oder durch die Nase geatmet wird. Falls der Nasenbereich z.B. durch Schnupfen verstopft ist, läßt sich diese Maske trotzdem noch uneingeschränkt nutzen.

Durch bedarfsmäßige Veränderung der Auslaßöffnung läßt sich diese Maske auch gut zur künstlichen Beatmung einsetzen. Hier darf jedoch nur mit zusätzlichen, geeigneten Meßgeräten gearbeitet werden.

Die Erfindung ist bestens geeignet für Rettungseinsätze in vergasten Räumen sowie unter Wasser. Der Einsatz dieser Maske eignet sich besonders bei unter Wasser eingeschlossenen Personen. Hier insbesondere, wenn es sich um mehrere Personen handelt. Es müssen nur, je nach Personenzahl, die Anzahl der Masken und nicht schwere und sperrige Sauerstoffflaschen mitgeführt werden. Aufgrund der Verbindung mittels des flexiblen Koaxialschlauchs mit dem Überdruckbehälter sowie der Abführung der ausgeatmeten Luft ist eine zeitlich unabhängige sowie weitest gehende Bewegungsfreiheit gewährleistet.

Um eine vollständige Unterwasserbehandlung vomehmen zu können, muß der Patient idealer weise vollständig und ohne Beckenrandberührung im Wasser liegen. Dies ist ohne eine Atemhilfe nicht möglich. Mit der Mund/Nase-Frischluft-Maske ist eine zeitlich uneingeschränkte Behandlung in absoluter Entspannungshaltung möglich. Wasser bzw. spezielle Flüssigkeiten bieten das ideale Entspannungsmedium. Ohne Atemmaske wäre der Aufenthalt unter der Oberfläche nur kurzzeitig möglich. Mit der Mund/Nase-Frischluft-Maske ist dies uneingeschränkt möglich. Mittels des flexiblen Koaxialschlauchs ist auch eine absolute Bewegungsfreiheit gewährleistet.

Die Krümmung eines in der Vorkammer vorgesehenen Rohres bewirkt, daß der Benutzer nicht durch unangenehme Luftströmung der Frischluftzufuhr (Zug) in der Entspannung beeinträchtigt wird.

Reparaturen und dergleichen unter Wasser waren derzeit nur mit einer Taucherausrüstung ausführbar. Dies hatte eine zeitliche Einschränkung zur Folge. Mittels der Mund/Nase-Frischluft-Maske ist nun ein Aufenthalt ohne sperrige Sauerstoffflasche und zeitlich unbegrenzt möglich.

Auch Siloanlagen bergen immer wieder die Gefahr, daß bei Kontrollgängen die Person unbeabsichtigt die tödlichen Gase einatmet und erstickt. Hier ist eine Mund/Nase-Frischluft-Maske die ideale Lösung. Auch für nachfolgende Helfer ist es unerläßlich, sich mit der Mund/Nase-Frischluft-Maske zu schützen. Aufgrund des Überdruckbehälters, wie z.B. einer handelsüblichen elektrischen Luftpumpe, die auch am Zigarettenanzünder des Fahrzeugs angeschlossen werden kann, ist die Mund/Nase-Frischluft-Maske universell einsetzbar.

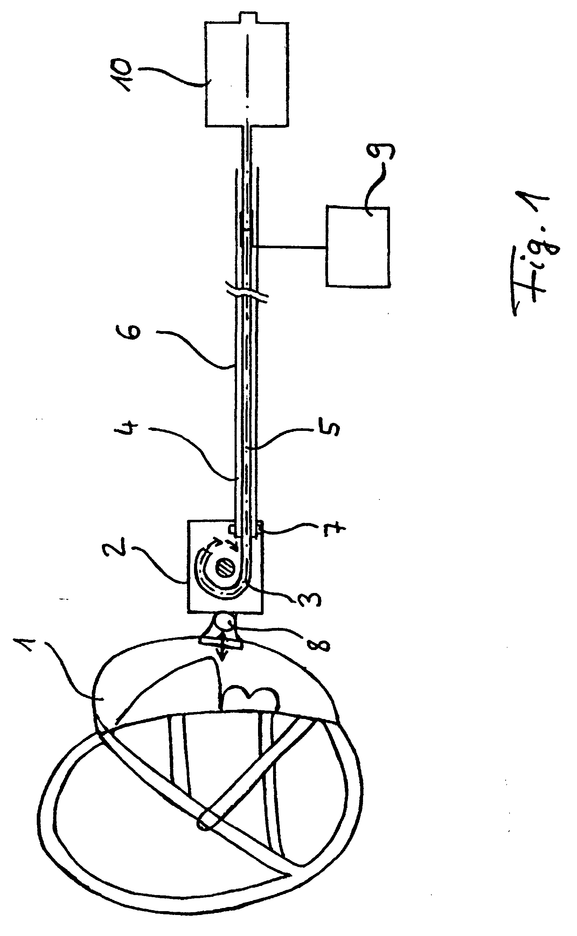

Die vorliegende Erfindung wird im folgenden an einer Ausführungsform anhand der beigefügten Zeichnung näher erläutert. Dabei zeigt
- Fig. 1: eine schematische Darstellung einer erfindungsgemäßen Mund/Nase-Frischluft-Maske

Die Mund/Nase-Frischluft-Maske besteht aus einer beispielsweise aus dem Anästhesiebereich bekannten Grundmaske 1. Diese Maske schließt vollständig fluiddicht um den Mund-Nasenbereich ab. Mittels Klettverschluß wird die Maske um den Kopf so fixiert, daß ein Verrutschen unmöglich ist. Bei Bedarf kann auch eine Gummihaube mit Augengläsern Verwendung finden. Die Öffnung der Maske führt in eine kleine Vorkammer 2. In dieser Vorkammer 2 wird die mit geeignetem Überdruck eingeblasene Frischluft aufgrund eines gebogenen Rohres 3 so verwirbelt, daß keine störenden Zugerscheinungen auftreten. Gleichzeitig ist dadurch gewährleistet, daß die ausgeatmete Luft vollständig durch einen Abluftschlauch 4, welcher äußerer Bestandteil eines Koaxialschlauchs 6 ist, nach außen befördert wird (Sogwirkung/Totraumventilation), so daß dem Benutzer immer absolute Frischluft zur Verfügung steht. Somit gibt es keine zeitliche Einschränkung hinsichtlich der Luftzufuhr.

Der Koaxialschlauch 6 besteht aus zwei ineinanderliegenden flexiblen Schläuchen (Frischluftschlauch 5, Abluftschlauch 4). Eine diesem ähnliche Ausführung ist in der Patentschrift DE 40 23 108 C1 beschrieben, weist dort jedoch den großen Nachteil auf, daß die Schläuche direkt mit der Atemmaske verbunden sind, was zu den oben genannten unangenehmen Nebenwirkungen des Luftstroms führt und eine Entspannung unmöglich macht.

Die Vorkammer 2 besteht aus zwei Halbschalen. In der einen Schale ist ein gebogenes Rohr für den Anschluß des Frischluftschlauchs befestigt. Die andere Schale dient als Deckel. Diese beiden Teile werden mittels Schrauben und Gummilippen fluiddicht verschlossen. Der Frischluftschlauch 5 wird in das gerade Ende des gebogenen Rohres 3 geschoben und mittels eines Dorns fixiert. Das Einschieben in das Anschlußstück dient auch zu dem Zweck, daß keine ausgeatmete Luft in den Frischluftschlauch 5 gerät und somit eine leichte Handhabung zur Desinfektion gewährleistet ist. Der äußere Abluftschlauch 4 wird mittels seiner Rippen in einer ebenfalls gerippten Umwandung 7 gelagert, um so ein Verrutschen wirksam zu verhindern.

Das Verbindungsstück der Vorkammer 2 zur Grundmaske 1 ist mit einem Abzweig 8 versehen, an den bei Bedarf medizinisches Gerät wie beispielsweise eine Magensonde oder dergleichen angeschlossen werden kann. Dieser Abzweig 8 ist bei Nichtbenutzung mittels eines Verschlusses deaktiviert.

Der vorzugsweise innere Schlauch 5 des Koaxialschlauchs 6 führt die Frischluft in geeignetem Überdruck. Dadurch ist gewährleistet, daß genügend Frischluft zum Benutzer transportiert wird und auch keine ausgeatmete Luft im Atembereich zurückbleibt.

Der zweite Schlauch 4, vorzugsweise der äußere, ist in der Dimension so gewählt, daß die ausgeatmete Luft und die überschüssige Frischluft ungehindert entweichen können. Die Öffnung des Abluftschlauchs 4 ist zweckmäßigerweise außerhalb der Flüssigkeitsoberfläche bzw. des zum Atmen ungeeigneten Mediums bzw. des Raumes der von ausgeatmeter Luft rein gehalten werden soll angeordnet. Damit ist gewährleistet, daß kein zum Atmen ungeeignetes Medium, auch während des Atemvorgangs, eindringen kann.

Aufgrund des flexiblen Koaxialschlauchs 6 ist ein versehentliches Abknicken oder Stoppen der Luftzu-/-abfuhr ausgeschlossen. Der flexible Koaxialschlauch 6 dient auch zur Sicherstellung einer maximalen Bewegungsfreiheit.

Der Überdruck wird je nach Bedarf mittels einer stationären Überdruckkammer 10 bzw. eines mobilen Überdruckgerätes, wie herkömmliche elektrische Luftpumpen, die meist ausreichend sind, in Verbindung mit einem Manometer geliefert. Bei normaler Nutzung von gesunden Personen kann auf das Manometer verzichtet werden, da die überschüssige Luft ungehindert entweichen kann.

Zu medizinischen und/oder therapeutischen Zwecken kann eine weitere Kammer 9 zur Luftbefeuchtung, zur Zugabe von medizinischen Zusatzstoffen oder dergleichcn zwischengeschaltet werden.

Für Einsätze im Feuerbereich, beispielsweise Rettung aus Brandherden, ist der flexible Koaxialschlauch 6 aus hitzebeständigem Material vorzusehen. Die Einspcisung der Frischluft erfolgt dann zweckmäßigerweise mittels vorgekühlter Luft, damit sie beim Abnehmer eine zum Atmen geeignete Temperatur aufweist. Im Kälteeinsatz ist entsprechend umgekehrt zu verfahren.

Aufgrund der Anordnung sind die Grundmaske 1 sowie die Vorkammer 2 zu Desinfektionszwecken und bei Nutzerwechsel leicht auswechselbar.

### Bezugszeichenliste

- 1 -: Grundmaske;
- 2 -: Vorkammer;
- 3 -: gebogenes Rohr,
- 4 -: Abluftschlauch;
- 5 -: Frischluftschlauch;
- 6 -: Koaxialschlauch;
- 7 -: Umwandung;
- 8 -: Abzweig;
- 9 -: Kammer;
- 10 -: Überdruckkammer.

## Patentansprüche

1. Atemmaske zur Versorgung von Personen mit insbesondere Frischluft über Mund und/oder Nase, umfassend eine vollständig fluiddichte Grundmaske (1), die mit einem flexiblen Koaxialschlauch (6) in Verbindung steht,
**dadurch gekennzeichnet,**
**daß** zwischen dem flexiblen Koaxialschlauch (6) und der Grundmaske (1) eine Vorkammer (2) angeordnet ist, wobei ein innerer Schlauch (5) des Koaxialschlauchs (6) mit einem in der Vorkammer (2) vorgesehenen gebogenen Rohr (3) verbunden ist und ein äußerer Schlauch (4) des Koaxialschlauchs (6) gegen die Vorkammer (2) abgedichtet ist und in diese mündet.

2. Atemmaske nach Anspruch 1, **dadurch gekennzeichnet, daß** der innere Schlauch (5) des Koaxialschlauchs (6) auf der nicht mit der Vorkammer (2) verbundenen Seite in einen Frischluftbereich mündet.

3. Atemmaske nach Anspruch 1 oder 2, **dadurch gekennzeichnet, daß** der äußere Schlauch (4) des Koaxialschlauchs (6) auf der nicht mit der Vorkammer (2) verbundenen Seite in einen vorzugsweise Frischluftbereich mündet.

4. Atemmaske nach einem der vorhergehenden Ansprüche, **dadurch gekennzeichnet, daß** die Atemmaske auf der dem Gesicht der Person zugewandten Seite wenigstens eine Dichtung aufweist.

5. Atemmaske nach einem der vorhergehenden Ansprüche, **dadurch gekennzeichnet, daß** die Atemmaske mit einem Überdruckgerät in Verbindung steht.

6. Atemmaske nach einem der vorhergehenden Ansprüche, **dadurch gekennzeichnet, daß** wenigstens ein Anschluß vorzugsweise im Bereich des Koaxialschlauchs (6) zur Zugabe von Zusatzstoffen in die Atemluft vorgesehen ist.

7. Atemmaske nach einem der vorhergehenden Ansprüche, **dadurch gekennzeichnet, daß** vorzugsweise zwischen Grundmaske (1) und Vorkammer (2) wenigstens eine verschließbare Öffnung (8) vorgesehen ist.

8. Atemmaske nach einem der vorhergehenden Ansprüche, **dadurch gekennzeichnet, daß** der äußere Schlauch (4) in einen Entkeimungsbehälter oder in einen Entkeimungsraum mündet.

9. Atemmaske nach einem der vorhergehenden Ansprüche, **dadurch gekennzeichnet, daß** der äußere Schlauch (4) in ein stationäres Entlüftungssystem mündet.

10. Atemmaske nach einem der vorhergehenden Ansprüche, **dadurch gekennzeichnet, daß** der innere Schlauch (5) des Koaxialschlauchs (6) vorzugsweise zur Frischluftzufuhr und der äußere Schlauch (4) vorzugsweise zur Abführung der verbrauchten Atemluft vorgesehen ist.

11. Verwendung der Atemmaske gemäß Anspruch 1 bis 10 als Unterwassermaske insbesondere zu medizinischen Zwecken in Flüssigkeiten.

12. Verwendung der Atemmaske gemäß Anspruch 1 bis 10 als Atemschutzmaske zum Schutz von Personen, welche sich in atmungsungeeigneten Medien oder für die Atmung ungeeigneten Arbeitsbereichen aufhalten.

13. Verwendung der Atemmaske gemäß Anspruch 1 bis 10 als Absaugmaske zum Abführen von Atemluft in Arbeitsbereichen, in denen die ausgeatmete Luft unerwünscht ist, insbesondere in Reinsträumen.

14. Verwendung der Atemmaske gemäß Anspruch 1 bis 10 als Absaugmaske zum Abführen von Atemluft von hochinfektiösen Patienten.

15. Verwendung der Atemmaske gemäß Anspruch 1 bis 10 als Unterwassermaske insbesondere für Freizeit und Erhohlung.

## Claims

1. Respiratory mask for supplying persons with, in particular, fresh air via the mouth and/or nose, comprising a completely fluid-tight base mask (1) which communicates with a flexible coaxial tube (6), **characterized in that** a prechamber (2) is arranged between the flexible coaxial tube (6) and the base mask (1), an inner tubing (5) of the coaxial tube (6) being connected to a bent pipe (3) provided in the prechamber (2), and an outer tubing (4) of the coaxial tube (6) being sealed off against the prechamber (2) and leading into the latter.

2. Respiratory mask according to Claim 1, **characterized in that** the inner tubing (5) of the coaxial tube (6) leads into a fresh air area on the side not connected to the prechamber (2).

3. Respiratory mask according to Claim 1 or 2, **characterized in that** the outer tubing (4) of the coaxial tube (6) leads into a preferably fresh air area on the side not connected to the prechamber (2).

4. Respiratory mask according to one of the preceding claims, **characterized in that** the respiratory mask has at least one seal on the side directed towards the person's face.

5. Respiratory mask according to one of the preceding claims, **characterized in that** the respiratory mask communicates with an overpressure device.

6. Respiratory mask according to one of the preceding claims, **characterized in that** at least one attachment for delivering additives into the respiratory air is provided preferably in the area of the coaxial tube (6).

7. Respiratory mask according to one of the preceding claims, **characterized in that** at least one closable opening (8) is provided preferably between base mask (1) and prechamber (2).

8. Respiratory mask according to one of the preceding claims, **characterized in that** the outer tubing (4) leads into a sterilizing container or into a sterilizing space.

9. Respiratory mask according to one of the preceding claims, **characterized in that** the outer tubing (4) leads into a stationary air removal system.

10. Respiratory mask according to one of the preceding claims, **characterized in that** the inner tubing (5) of the coaxial tube (6) is preferably provided for supplying fresh air and the outer tubing (4) is preferably provided for removing the used respiratory air.

11. Use of the respiratory mask according to Claims 1 to 10 as an underwater mask, in particular for medical purposes in liquids.

12. Use of the respiratory mask according to Claims 1 to 10 as a protective breathing mask for protecting persons who are present in media not suitable for breathing or in work areas not suitable for breathing.

13. Use of the respiratory mask according to Claims 1 to 10 as an extraction mask for removal of respiratory air in work areas where the exhaled air is undesirable, particularly in clean-room environments.

14. Use of the respiratory mask according to Claims 1 to 10 as an extraction mask for removal of respiratory air from highly infectious patients.

15. Use of the respiratory mask according to Claims 1 to 10 as an underwater mask particularly for leisure and recreation purposes.

## Revendications

1. Masque respiratoire pour l'approvisionnement particulièrement en air frais de personnes par la bouche et/ou par le nez, comprenant un masque principal (1) entièrement étanche aux fluides qui est relié à un tuyau coaxial flexible (6), **caractérisé en ce qu'**une préchambre (2) est disposée entre le tuyau coaxial flexible (6) et le masque principal (1), un tuyau intérieur (5) du tuyau coaxial (6) étant relié à un tube (3) recourbé prévu dans la préchamhre (2) et un tuyau extérieur (4) du tuyau coaxial (6) étant étanche, par rapport à la préchambre (2) et débouche dans celle-ci.

2. Masque respiratoire selon la revendication 1, **caractérisé en ce que** le tuyau intérieur (5) du tuyau coaxial (6) débouche dans une zone d'air frais sur le côté qui n'est pas relié à la préchambre (2).

3. Masque respiratoire selon la revendication 1 ou 2, **caractérisé en ce que** le tuyau extérieur (4) du tuyau coaxial (6) débouche de préférence dans une zone d'air frais sur le côté qui n'est pas relié à la préchambre (2).

4. Masque respiratoire selon l'une quelconque des revendications précédentes, **caractérisé en ce que** le masque respiratoire présente au moins un élément d'étanchéité sur le côté tourné vers le visage de la personne.

5. Masque respiratoire selon l'une quelconque des revendications précédentes, **caractérisé en ce que** le masque respiratoire est relié à un appareil de surpression.

6. Masque respiratoire selon l'une quelconque des revendications précédentes, **caractérisé en ce qu'**au moins un raccord, de préférence dans la zone du tuyau coaxial (6), est prévu pour l'ajout de substances additionnelles dans l'air à respirer.

7. Masque respiratoire selon l'une quelconque des revendications précédeutes, **caractérisé en ce qu'**au moins une ouverture refermable (8) est prévue de préférence entre le masque principal (1) et la préchambre (2).

8. Masque respiratoire selon l'une quelconque des revendications précédentes, **caractérisé en ce que** le tuyau extérieur (4) débouche dans un récipient de stérilisation ou dans un espace de stérilisation.

9. Masque respiratoire selon l'une quelconque des revendications précédentes, **caractérisé en ce que** le tuyau extérieur (4) débouche dans un système d'aération stationnaire.

10. Masque respiratoire selon l'une quelconque des revendications précédentes, **caractérisé en ce que** le tuyau intérieur (5) du tuyau coaxial (6) est prévu de préférence pour l'alimentation en air frais et le tuyau extérieur (4) est prévu de préférence pour l'évacuation de l'air vicié.

11. Utilisation du masque respiratoire selon les revendications 1 à 10 comme masque subaquatique dans des liquides en particulier dans un but médical.

12. Utilisation du masque respiratoire selon les revendications 1 à 10 en tant que masque respiratoire de protection pour protéger des personnes qui se trouvent dans des milieux inappropriés à la respiration ou dans des zones de travail inappropriées à la respiration.

13. Utilisation du masque respiratoire selon les revendications 1 à 10 comme masque d'aspiration pour l'évacuation d'air respiré dans des zones de travail où l'air expiré est indésirable, en particulier dans des salles blanches.

14. Utilisation du masque respiratoire selon les revendications 1 à 10 comme masque d'aspiration pour l'évacuation d'air respiré de patients extrêmement contagieux.

15. Utilisation du masque respiratoire selon les revendications 1 à 10 comme masque subaquatique en particulier pour les loisirs et la détente.
